# EUROPEAN PATENT SPECIFICATION

(11) **EP 0 965 043 B1**
(45) Date of publication and mention of the grant of the patent: **10.04.2002**
(21) Application number: 97901090.7
(22) Date of filing: 14.01.1997
(51) Int. Cl.: G01N 33/532, G01N 33/68

(54) **DETECTION OF CARDIAC MUSCLE NECROSIS BY IMMUNOASSAY AND APPROPRIATE ANTIBODIES THEREFOR**
ENTDECKEN VON NEKROSEN DES HERZMUSKELS DURCH EINEN IMMUNOASSAY UND ENTSPRECHENDE ANTIKORPER
DETECTION DE LA NECROSE DU MUSCLE CARDIAQUE A L'AIDE D'IMMUNODOSAGES ET D'ANTICORPS APPROPRIES A CET EFFET

(30) Priority: 15.01.1996 FI 960167
(43) Date of publication of application: 22.12.1999
(73) Proprietor: Hytest Oy, 20520 Turku (FI)
(72) Inventor: KATROUKHA, Alexei, Moscow (RU); ESKOLA, Jarkko, FIN-20610 Turku (FI); SEVERIN, Eugenii, Moscow (RU); SEVERIN, Sergey, Moscow (RU); KORPELA, Timo, FIN-20500 Turku (FI)
(74) Representative: Öhman, Ann-Marie
(86) International application number: FI9700016
(87) International publication number: WO9726534

(56) References cited:
- WO-A-96/33415
- DE-A- 4 405 249
- LABORATORY MEDICINE, Volume 23, No. 5, May 1992, HUGO A. KATUS et al., "Proteins of the Troponin Complex", page 311.

## Description

### FIELD OF INVENTION

This invention relates to clinical diagnostics and more particularly to the diagnostics and therapy follow-up of heart muscle necrosis. The invention concerns with a new approach to monitor the condition of heart by leakage of two proteins, troponin I and C, from necrotic heart muscle.

### BACKGROUND OF THE INVENTION

Heart infarction is one of the commonest causes of death of adult persons in developed countries. In case there is available quick diagnosis after infarction followed by appropriate medicinal treatment or surgical operation, a significant part of the patients can be rescued. The required laboratory diagnostics is traditionally based on the detection of elevated enzyme activities of e.g. creatinine kinase (CK), lactate dehydrogenase (LD), or aspartate aminotranferase (GOT) in serum. The drawback is, however, the ambiguousness of interpreting the results since the enzymes can also originate from organs other than heart. Hence, the diagnostics of the acute myocardial infarction (AMI) is to be based on the laboratory tests accompanied with rather diffuse clinical symptoms or on electrocardiography (ECG).

To achieve reliable diagnosis of heart infarction, the nonspecificity of the customary laboratory tests brings about the need for a few sequential samples as a function of time. This results in a slowness of getting the final diagnosis that is very severe drawback for the required therapeutic actions (for a review, see Journal of Clinical Immunoassay, Vol. 17, No.1, pp.6-48). An objective for future is to diagnose infarction by one serum sample only. This would require a reliable marker which can be measured in high accuracy. The resulting value can be interpreted statistically to reconstruct the history of the infarction.

The building block proteins of the heart muscle contractile apparatus have been subject of intensive studies for their applicability to the heart infarction diagnostics (F.C. Apple: Laboratory Medicine, May 1992, Vol. 23, No. 5, pp. 311-317). The troponins are proteins located on the thin film filament of the muscle contractile apparatus. There are three troponins (Tns), troponin T (tropomyosin-binding subunit with molecular mass of 39kD), Tn I (actomyosin-adenosine triphosphatase-inhibiting subunit with molecular mass of 26.5kD), and Tn C (calcium-binding subunit with molecular mass of 18kD), acting together to regulate the muscle contraction. The heart Tns, excluding Tn C, are immunologically different from those in skeletal muscle. This forms the grounds of utilizing troponins as specific markers of heart muscle damages.

Recently some diagnostic kits for heart Tns I and T have become commercially available. It has been thought that cardiac Tn C is not suitable for heart diagnostics at all since the slow-twitch skeletal isoform of Tn C is identical to the cardiac isoform. Tn I also exists in cardiac, fast-twitch and slow-twitch skeletal isoforms. The cardiac isoform of Tn I is about 40% dissimilar from the skeletal isoform and contains an additional specific functional amino acid sequence on the N-terminus. Despite of the instability of Tn I, it is considered as such one of the most promising markers of the cardiac injury (SCRIPPS NEWS, CA, USA, 1993, Vol. 7, No.2.).

DE 4405249 (Larue & Marquet) discloses stabilized composition of troponin I or T for immunoassays, comprising an aqueous solution or powdered preparation containing troponin I ortroponin T, 1 to 10 molar equivalents of troponin C per molar equivalent of troponin I or T, and optionally Mg²⁺ and/or Ca²⁺ ions.

### SUMMARY OF INVENTION

The object of this invention is an improved method for determination of the status of cardiac muscle necrosis by immunoassay comprising of simultaneous determination of two cardiac muscle troponins I and C (Tn I and Tn C), able to bind to each other to form a complex I-C. The invention exploits the observation that variable portion of Tn I in serum is complexed with Tn C. The complexed Tn I (I-C) and Tn I - complexable to externally added Tn C (total Tn I-C), or the ratio Tn I-C/total Tn I-C, can be used as an improved measure for heart infarction diagnostics.

### BRIEF DESCRIPTION OF THE DRAWINGS AND TABLES

Figure 1: Gel chromatography of Tn I added to a normal serum and of a serum of a patient with acute myocardial infarction (AMI) on a column separating proteins according to their molecular masses from 10,000 to 1 million daltons (calibration points with known protein standards indicated).

Figure 2: Gel electrophoresis pattern of Tns C, I, and T on gradient polyacrylamide gel (10-20% acrylamide). Figure 2 shows that Tn I exists in serum of AMI patients in a complex mainly with Tn C.

Figure 3: Analysis of normal serum and three sera from suspected AMI patients (AMI Sample 1-3) by using different monoclonal antibodies. NHS refers to Normal Human Serum.

Figure 4: Figure 4 shows that when pure Tn I is added to normal serum (all-male serum) the signal increases indicating that normal serum pool contains Tn C. External Tn C increases signal dramatically whenever Tn C is detected with a labeled Tn C-specific antibody (here clone 1A2). SA refers to Streptavidin coated onto a microtiter plate surface and B to Biotin.

Figure 5: Mesurement of Tn I-C complex in sera of 11 potential AMI patient after hospital admission by a sandwich assay (coating antibody 8E10 and labeled antibody 1A2). Black bars refer to Tn I-C and grey bars to total Tn I-C complex obtained by adding Tn C (1 µg/microtiter plate well).

Figure 6: Correlation between Tn I and Tn I-C complex (10F4 coating and 7F5 labeling for Tn I; 8E10 coating and 1A2 labeling for Tn C) in sera of potential AMI patients sampled in different times from the infarction. The values of the large squares are not included in the regression line. They represent exceptional values in the very beginning and the late stage of infarction.

Figure 7: Variation of signal from Tn I (10F4 coating, 7F5 labeled) and Tn I-C (8E10 coating, 1A2 labeled) as a function of time in sera of AMI patients.

Figure 8: Signal obtained out of Tn I-C disassembled to obtain Tn I by addition of EDTA (antibodies 10F4 and 1A12) (Example 2)

Table I: Correlation coefficient between measurement of troponin I (by methods I1 and I2), Tn I-C, total Tn I-C, CK, and CKB.

### BROAD DESCRIPTION OF THE INVENTION

Troponins T, I, and C are immobilized together as a group in the muscle contractile apparatus. Troponin T is found to contain an unbound cytosolic pool of 6% of total myocardial Tn T content. Washout of this pool from muscle cells may cause the peak of Tn T concentrations in serum on the first day of early reperfusion of the infarct zone. There is a rapid disappearance of Tn I from myofibrils during myocardial infarction but Tn T was shown to persist in the zone nearly unaltered for six days. The degradation rate of Tn T is comparable to that of myosin light chains. The difference in the disappearances of Tns I and T from myofibrils may account for the differences in the release kinetics of both markers in patients with nonperfused myocardial infarction. The continuous degradation of myofibrils during infarct evolution and healing results in a resistant egress of myofibril components into circulation (Katus, H.A., et al. in Laboratory Medicine, Vol. 23, 1992, p.312).

As stated above, the release kinetics of Tns into serum from necrotic zone of heart is complicated. Tn C is not considered useful as the marker of myocardial infarction, whereas Tn I is for a long time considered as one of the most promising specific markers.

According to this invention a significant, but variable, portion of Tn I in serum of AMI patients exists as a complex of Tn I with Tn C (Tn I-C) bound with other proteins and this Tn I-C is used for heart infarction diagnostics either as such or so that external Tn C is added (total I-C). Additional advantages are obtained from concomitant stabilization of Tn I by Tn C and in this way Tn I content in serum is standardized resulting in a more accurate and reproducible measure for the infarction.

Scheme 1 illustrates the principal reactions which the present invention is based onto.

According to their biological function, the molecular conformations of each Tns and their mutual interaction change considerably during the contraction and release of muscle tension. Concomitantly the immunological properties of Tns and especially that of Tn I shall be different when it is free or bound to Tn C. If the sample contains both free Tn I and complex Tn I-C, the primary antibody binds different portion of free Tn I and Tn I-C complex. It is shown in this invention that individual patient samples contain fairly different ratios of free and complexed Tn I. Thus, the measurement of only Tn I does not necessarily probe the real amount of Tn I. If the second labeled antibody also binds to Tn I, the complication arising from changed specificities along with conformational changes is still amplified. A further difficulty originates from the fact that free Tn I contains hydrophobic areas in the molecule that cause its strong nonspecific binding to various materials including glass and plastics. This area locates most probably at the Tn I-C interface or the Tn I-C interaction otherwise affects occurrence of such areas. Since different serum samples contain different ratio of Tn I-C/ total Tn I-C contents, and since the determination of exact nonspecific binding background in the signal is tedious, it is highly desirable to stabilize the serum samples in the respect of these background effects. In conclusion, the ratio Tn I-C /total Tn I-C includes considerably more information about the history and degree of damage of the heart infarction than measurement of Tn I.

Pure added Tn I is very unstable molecule in serum and in other solutions. Its immunological activity decreases to 20% within one hour at 37°C. British Patent Application GB 2 275 774 A claims that Tn I standard preparation for immunoassays can be stabilized by addition of Tn C and optionally Mg⁺⁺- or Ca⁺⁺ -ions. However, the cited invention did not exploit the stabilizing effect of Tn I by Tn C being released from heart, skeletal muscle, or added from an external source.

In the present invention a related stabilization as with the cited Tn I standard preparation above, is exploited for increasing the reproducibility and sensitivity of Tn I assays. Such external Tn C can originate from any source including those produced in microbes by the recombinant techniques. The stabilization also provides an additional advantage since the clinical samples may be stored for an undetermined periods before the measurement. Such stabilization can be carried out by adding Tn C into blood sampling tube, to serum, or to a sample to be analyzed.

### DETAILED DESCRIPTION OF THE INVENTION

The antibodies mentioned in this invention refer to those found in the catalogs of Hytest Ltd., Turku, Finland or BioSpacific Ltd, California, USA.

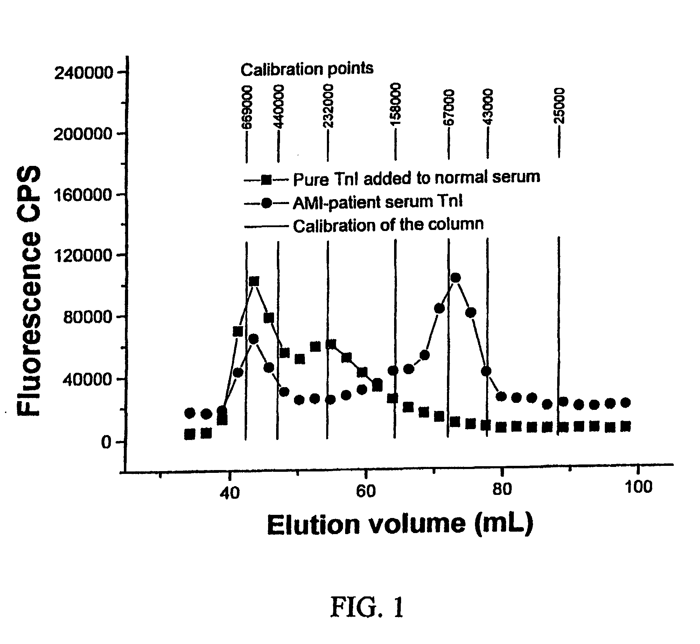

In the present invention, Fast Protein Liquid Chromatography (FPLC) technique with a gel chromatography column having molecular mass separation range of 10kD- 1000 kD was applied in non-denaturating conditions (Figure 1). The profile of proteins as measured by absorbance at 280 nm in AMI patient samples does not differ from normal serums; three major peaks with molecular masses of about 65 kD, 160 kD and 1000 kD appeared with both (not shown in Figure 1).

Figure 1 shows that any of the Tns does not exist in the serum in free form but are bound to other biomolecules and usually separated into two fractions in the chromatographic conditions. Also pure Tn I added to normal serum, not containing heart-originated proteins, rapidly form the aggregates.

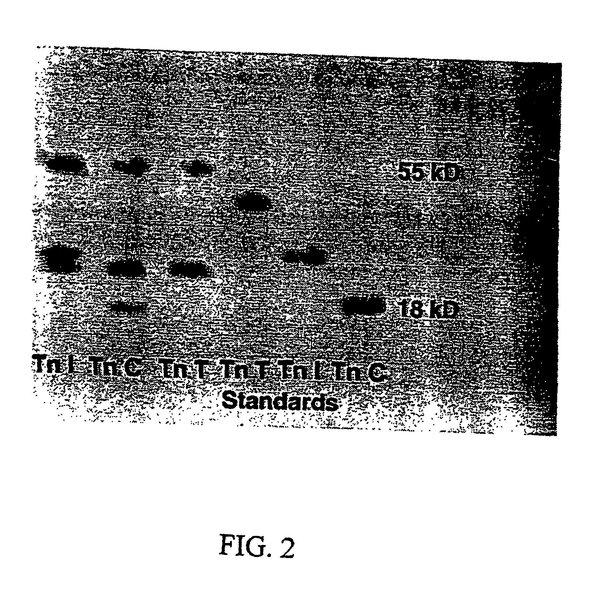

Figure 2 shows that when Tns I, C, and T are captured by an immunoaffinity adsorbent from serum pool of AMI patients, followed by washing and eluting out of the adsorbent at low pH, only a complex containing of Tns I and C are detected. Thus Tn T does not significantly bind to Tn I-C.

Figure 2 shows pure standard Tns, I, C, and T specifically detected on an electrophoresis gel. The three lanes left side represents electrophoresis of a sample of a serum from a pool of AMI patients also specifically detected by Tn I, Tn C, and Tn T -specific antibodies. The serum from AMI patients was first applied on an immunoaffinity column of Sepharose 4 B CL derivatized with monoclonal antibody known to bind the complex Tn T-I-C. This fraction was eluted from the column and subjected to the electrophoresis followed by the Western blotting. The membrane was allowed to react with specific mice antibodies against each individual Tn forms and stained thereafter with goat anti-mouse IgG labeled with horseradish peroxidase which was detected by chemiluminescence. The bands at the molecular masses at 55 and 25 kD originate from the heavy and light chains of the mouse antibodies used to capture the complex Tn I-C-T being partly detached from the affinity column by the acid eluent.

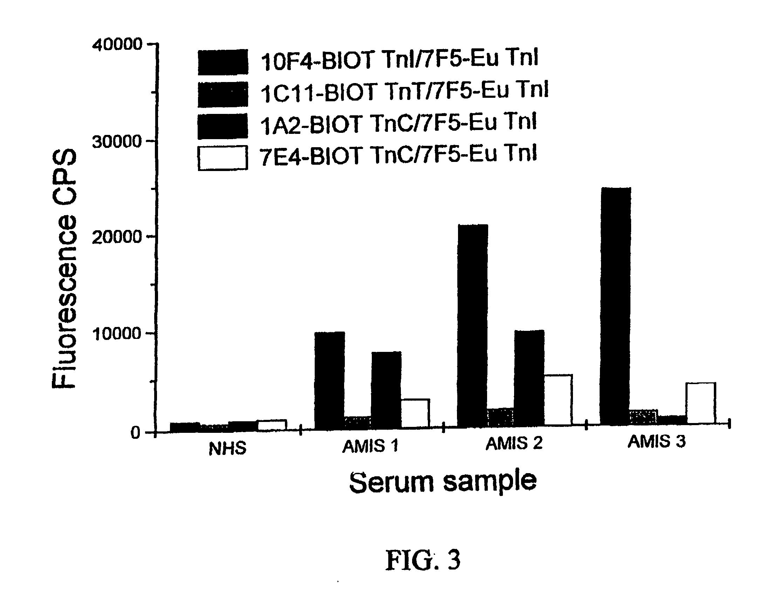

Figure 3 characterizes the test system of the present invention by different antibodies. The secondary labeled antibody (7F5) was specific to Tn I. The low signals with anti-Tn T antibody (1C11) as the primary catching antibody indicates that Tn T is not involved in complex with Tn I whereas anti-Tn C antibodies (1A2 and 7E4) yield remarkable signals. The antibody 10F4 is not strictly specific to Tn I but preferably recognizes Tn I in complex I-C.

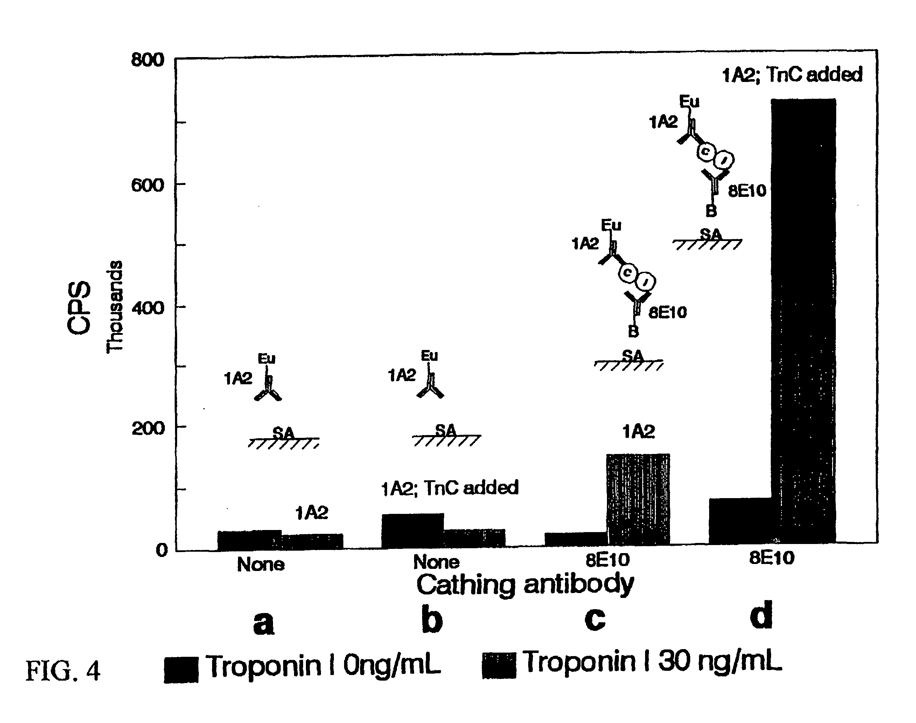

Figure 4 shows that serum pool of healthy blood donator men (all-male serum) contains Tn C which can be found and quantitated in the serum by adding Tn I purified from human heart. This proves that Tn C itself is a poor marker of heart muscle damages. If the catching antibody for Tn I is omitted in the test system, the background signals from the secondary, labeled antibody (1A2 specific to Tn C) are small and are even decreased by adding Tn I in concentration of 30 ng/ml (black bar in Figure 4 b). If Tn C is added into the same system, the background due to adsorption of Tn C onto the microtiter plate surface is slightly increased (grey bar in Figure 4 b) while addition of Tn I decreases it (black bar). In Figure 4 c, Tn I catching antibody is immobilized and pure Tn I (30 ng/ml) is added into normal serum (black bar). Since the background (grey bar) is small, and the other background controls are valid (see before), the normal serum contains Tn C. If a large excess of Tn C is added into the system similar to Figure 4 c, the signal increase (black bar) is dramatic while the background keeps still relatively small (Figure 4 d).

As is generally known in the immunological test systems related to those of experiment of Figure 4 d, addition of Tn C in increasing amounts, will increase the signal only to a certain point and thereafter the signal will decrease due to the so-called hook-effect. It can be avoided by employing sequential incubations with Tn C and secondary antibody with washing the wells between the incubations.

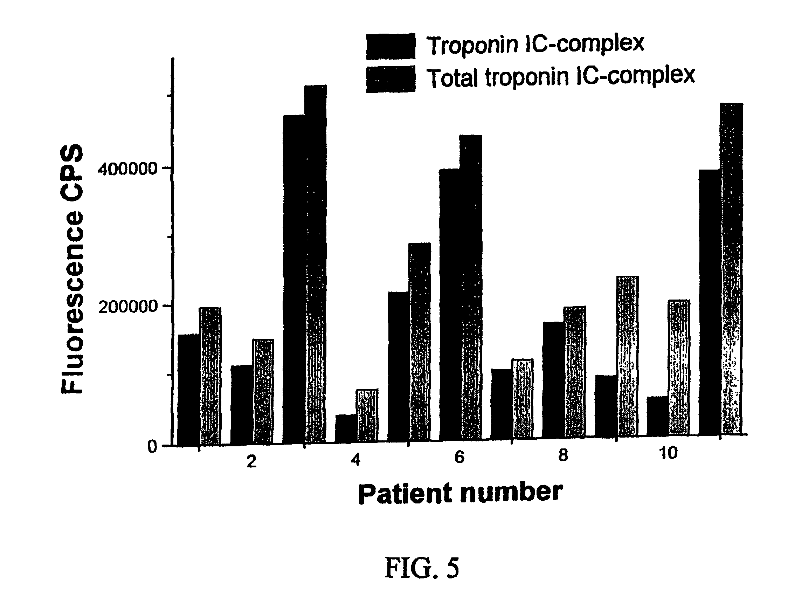

Figure 5 exemplifies the effects of externally added Tn C into serum samples of 11 potential AMI patients with high CK values. In all cases the signal increased by adding Tn C into the system being attributable to the additional formation of complex Tn I-C. The increase is not constant but considerably varies from patient to patient. The signal without addition of Tn C (Tn I-C complex in the serum; grey bars) compared to the signals after adding Tn C in excess (total Tn I-C, black bars) are around 75% in the average. This value can change accordingly with the experimental conditions.

Free Tn C originating from skeletal muscle may interfere with the values obtained for Tn I-C complex from heart in the system similar to Figure 5, where Tn I is captured by heart-specific antibody. The situation can be illustrated by two patients, one having heart attack after physical exercise and the other one without it. Both are assumed to have equal size of the necrotic zone. After the physical exercise free Tn C leaked from skeletal muscles, shall stabilize the Tn I leaking from heart and thus the increase by addition of external Tn C into such serum will be less than with the other one. On the contrary, the patient without Tn C leakage from skeletal muscle may have lower levels of measurable Tn I since it can have been reversibly denatured in the lower concentrations of stabilizing Tn C leaking from heart muscle only. However, in the latter case the signal increase within adding external Tn C can be considerably higher. Hence, the determination of Tn I-C and total Tn I-C values can yield extra information about the condition of the patient.

Table 1 shows results obtained by statistical treatment of the correlation coefficients of diagnostic results randomly chosen among AMI patients taken into hospital. It shows that correlation coefficients are usually highest in the cases the total Tn I-C, or when complex Tn I-C, or when Tn I-C (marked I+IC in Table 1) is measured by two labeled antibodies, one preferably binding to Tn C and the other one to Tn I. Total Tn I-C complex shows the best correlation to CK and CKB while only poor correlation occurs with the methods where CK and CKB are compared with Tn I measured by two different sandwich assays with two antibodies against Tn I (marked I1 : 7F5 coating, 10F4 labeled; and I2 : 8E10 coating, 7F5 labeled).

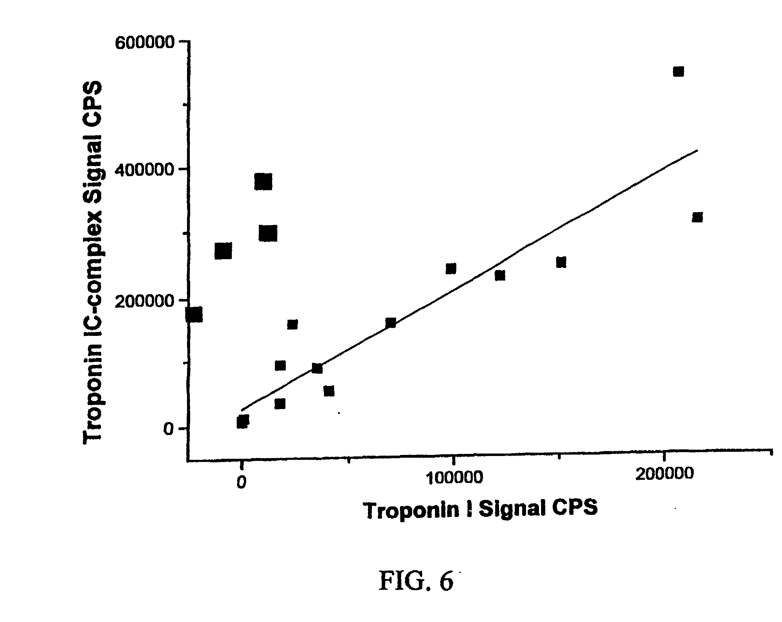

Figure 6 shows that generally there is a correlation between Tn I and complex Tn I-C but at early and late stages after hospital admission there appear large variations from the regression line. It is noticeable that this effect can be utilized to evaluate the history of infarction.

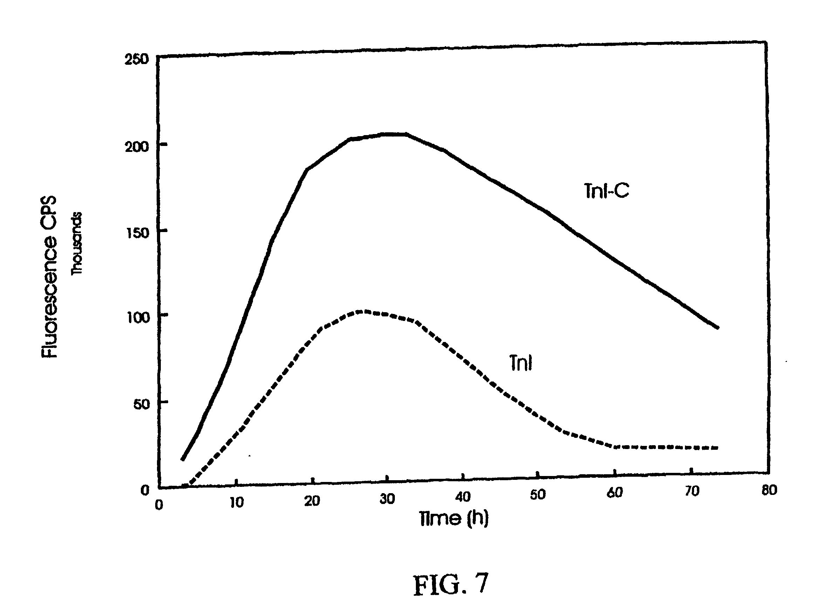

Figure 7 illustrates further the advantages of this invention by showing that the time-courses of the increase of the signal from Tn I (dashed line with using antibodies 10F4, 7F5) and complex Tn I-C (solid line with using antibodies 8E10, 1A2) are related in form but the signal from Tn I-C is much higher thus providing considerably higher sensitivity of the assay or earlier diagnosis.

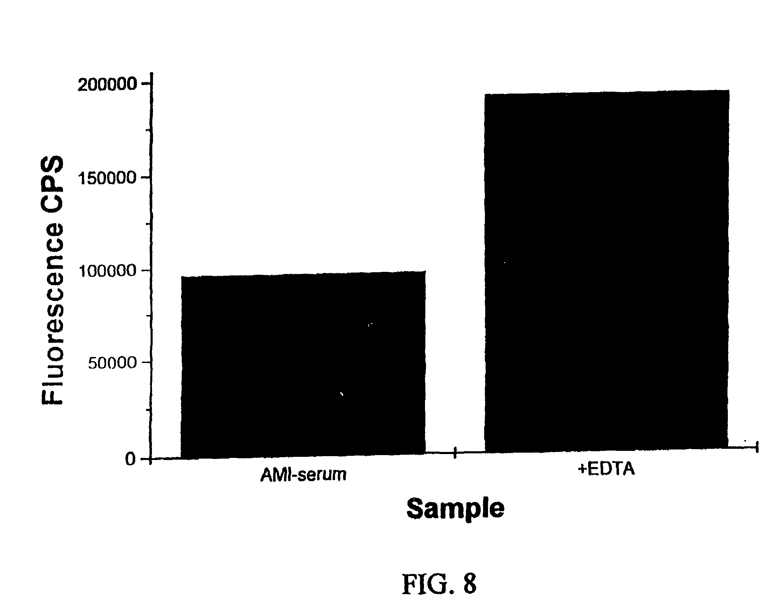

Figure 8 shows that addition EDTA increases the signal when the complex Tn I-C is detected by antibody pairs 10F4 and 1A12 (coating and labeled, respectively). Since the antibody 1A12 binds to Tn I and the signal from it increases by adding EDTA, the epitope of this antibody must situate near the mutual binding site of Tn I and Tn C. This provides another route to measure the total Tn I-C.
An additional advantage of the present invention is achieved by measuring the ratio of the complex Tn I-C in the presence or absence of EDTA or another complexing agent such as EGTA, DTPA, or citrate. Such treatment will destroy the Tn I-C complex as described in the British Patent Application GB 2200 358 A. In case the catching antibody is specific to Tn I, it is possible to measure the free and Tn I-C complex - releasable Tn C. If the secondary antibody has the same binding site as Tn C, it will measure free Tn I or Tn I unveiled from complex Tn I-C by EDTA.

Although apparently the system described above allows to measure the ratio of free Tn I / Tn I-C complex, there remains a drawback of nonspecific binding of Tn I to majority of materials used in the normal assays, including glass and plastics. It is, however, presumable that suitable materials can be found which do not absorb Tn I in nonspecific manner by selecting other materials or by presaturating normally used polystyrene or glass with appropriate compounds like proteins, polyvinylalcohol, polyvinylpyrrolidone, chitosan, carboxymethyl cellulose, gelatinized starch, or other related material.

The main embodiment of the present invention is that while Tn C is not useful for heart infarction diagnostics for its unspecificity, as such, it can be exploited indirectly for such diagnostics in the following ways: 1) by exploiting the observation that a considerable, but largely variable, portion of Tn I exists as a complex with Tn C in serum of AMI patients and by utilizing the ratio Tn I-C / total Tn I-C or Tn I-C - complexable with Tn C as a measure for heart muscle necrosis, 2) by stabilizing Tn I from denaturation with an external Tn C added into the sample, 3) by standardizing the signal/background ratio in different serum samples by driving Tn I totally to the stable complex Tn I-C, 4) by measuring the ratio Tn I-C/ Tn C - detachable from the complex Tn I-C, by measuring Tn I with and without addition of a strong complexing agent, such as EDTA in the serum sample, 5) by measuring simultaneously Tn I-C and total Tn I-C in such a manner that Tn I-C is measured by a specific antibody with label 1, and total Tn I-C is measured by a second antibody bound with troponin C and labeled with another label 2. Alternatively Troponin C is labeled with label 2. Labels 1 and 2 are preferably measured simultaneously at different wavelengths enabling to get the ratio Tn I-C / total Tn I-C in the same condition. Alternatively, the first and second labels can be other detectable molecule such as different enzymes, radioactive atoms, luminescence molecules, or fluorescing molecules with different wavelengths of detection. The complex Tn I-C can be also measured by a competitive assay where a labeled Tn I-C is added into a sample and a competition for the sample Tn I-C with the labeled Tn I-C is created. The binding antibody for the complex can be prepared from a polyclonal antibody through a proper fractionation procedure or by preparing a specific monoclonal antibody.

While the experimental studies herein are performed with a time-resolved techniques using fluorescence from europium ion, they are not by no means limited to this special technique. Equally well similar diagnostic methods can be performed with other usually available methods like ELISA with using any enzyme label, luminescence, radioactive or any other detection known in the prior art.

The invention is further illustrated by the following nonlimiting examples.

### EXAMPLE 1

### A time-resolved fluoroimmunoassay of Tn I-C and total Tn I-C

a) Labeling of monoclonal antibodies with europium chelate
   N¹-(p-isothiocyanatobenzyl)-diethylenetriamine-N¹,N²,N³,N³-tetraacetic acid europium complex (DTTA) (Wallac, Turku, Finland) in a 60-fold molar excess is allowed to react with the appropriate antibody at pH 9.8 overnight. The labeled antibody is separated from excess of DTTA on a column of Sephadex G-50 (1x5 cm) and Sepharose 6 B (1x50 cm) by using 50 mmol/L Tris-HCl buffer, pH 7.8, containing 9 g/L of NaCl and 0.05 % NaN₃ as the eluting agent (TSA buffer). Bovine serum albumin (1 g/L) is used as stabilizer in the solution.
b) Biotinylation of monoclonal antibodies
   The monoclonal antibody in concern (for example, 8E10) is allowed to react with 20-molar excess of an isothiocyanatophenyl derivative of trioxyethylene-amidibiotin (SCN-biotin) in 50 mmol/L sodium carbonate buffer, pH 9.8, for 2 h at the room temperature. The final concentration of the antibody in 1 mL of reaction mixture shall be around 1 mg/mL (described in ref. Hemmilä I, Heikkilä J, Leivo P. Direct and indirect immunofluorometry of thyrotropin [Abstract 671].Clin Chem 1989; 35:1206). The biotinylated antibody is separated from the reaction mixture by gel filtration using NAP-10 and PD-10 disposable columns (Pharmacia, Uppsala, Sweden) using TSA buffer as the eluent. Bovine serum albumin (1 g/L) is used as stabilizer in the solution .
c) Assay procedure

The time-resolved assay of Tn I or Tn I-C is performed with the streptavidine-coated microtiter wells (Wallac, Turku, Finland). Total Tn I-C assay is performed essentially similarly as Tn I and Tn I-C assays. Patient serum samples (or standards prepared in normal human serum) are added in the volume of 50 µL in to the wells of microtiter. Biotinylated catching antibody (300 ng/well), Eu-labeled antibody 200 ng/well, and in the case of measurement of total Tn I-C, Tn C 50 -2000 ng/well are added in TSA buffer containing 0.1 % bovine serum albumin (Wallac, Turku, Finland). The final reaction volume is 100 µL. After incubation for 1 h with gentle agitation at the room temperature, the strip wells are washed six times with Wash solution and 200 µL of Enhancement solution/well (Wallac, Turku, Finland) are added. After 5 min incubation with shaking and after a further 10 min without shaking the fluorescence is measured with a 1234 Delfia Research Fluorometer (Wallac, Turku, Finland).

### EXAMPLE 2

### Measurement of Tn I-C with and without EDTA

Microtiter plates are coated by with an antibody (10F4, Hytest, Turku, Finland) in 50 mmol/l sodium carbonate buffer, pH 9.2, at a concentration of 10 µg /mL overnight. The plate wells are washed six times with a wash solution (Labmaster Ltd., Turku, Finland) and saturated with bovine serum albumin. The secondary antibody (1A12, BiosPacific, USA) is labeled with europium chelate according to the instructions from the manufacturer (Wallac Ltd., Turku, Finland). The assay is carried out by adding 25 µL serum to the coated well and then 75 µL of assay buffer (Labmaster Ltd.) containing either 10 mM EDTA or not. After one hour incubation the wells are washed six times with the wash solution (Labmaster Ltd., Turku, Finland) and then the labeled antibody (200 ng/well) is added in the volume of 100 µL in the assay buffer (Labmaster Ltd.). After 1 hour incubation the wells are washed six times with the washing solution and 200 µL of Enhancement Solution (Wallac Ltd., Turku, Finland) is added and the fluorescence is measured after 10 minutes with a gentle shaking of the plate.

Ten serum samples of suspected AMI patients with elevated values of creatinine kinase B (from Central Hospital of the University of Turku) were analyzed. The average signal without EDTA was 96,000 cps and with EDTA 190,000 cps (Figure 8).

### EXAMPLE 3

### Measurement of Tn I-C by a competitive assay

A polyclonal antibody raised against human Tns I and C complex in mice is purified by immunoaffinity. Tn C is immobilized into the cyanogen bromide activated agarose according to the manufacturer's instructions (Pharmacia, Uppsala, Sweden) by using 1 mg of Tn C per ml of moist activated gel. Similar gel is prepared for Tn I. The crude antibody solution (1 mL ascites fluid) is eluted through both 1-mL columns in buffered (pH 7.8) physiological salt solution. The proteins eluted through the both columns are adsorbed onto an immobilized Tn I where Tn C is allowed to complex (Tn I-C column). The protein obtained from Tn I-C column is coated onto a microtiter plate by passive adsorption (Tienhaara et al., Clin.Chem. (1990), 36/11, 1961) (100 µg/well, overnight) and the wells are washed six times with a Wash solution (Wallac Ltd., Turku, Finland). Tn I-C complex is labeled with europium chelate (Wallac Ltd., Turku, Finland) as the antibody in Example 1. Sample with a constant known amount of labeled Tn I-C are added in the assay buffer (Wallac Ltd., Turku, Finland) into the microtiter wells as in Example 1. The incubations, washings, and measurements are thereafter performed essentially as in Example 1. The content of Tn I-C is inversely proportional to the signal obtained from labeled Tn I-C.

### EXAMPLE 4

### Measurement of Tn I-C and total Tn I-C by a double labeling technique.

The assay of Tn I-C is performed as described in Example 1 until the second labeled antibody specific to Tn C (e.g. 1A2) is added into the reaction mixture and incubated and washed six times. After this procedure a new reaction is carried out in the same wells within the reaction mixture with another labeled antibody, such the antibody 7F5 which binds to another epitope than the primary antibody on Tn I, or Tn C is labeled with another lanthanide chelate than europium; such as samarium, terbium, or yttrium. This labeling reaction can be performed essentially as the one with the europium chelate as described in Example 1 or in reference, Pettersson et al., (1992) Clin.Chem. 38/10, 2038-2043. Alternatively, both labeled antibodies are added in the same time and measured with a time-resolved fluorometer (wallac Ltd., Turku, Finland) within the Enhancement solution by fluorescence at the different wavelengths from the separate metal ions.

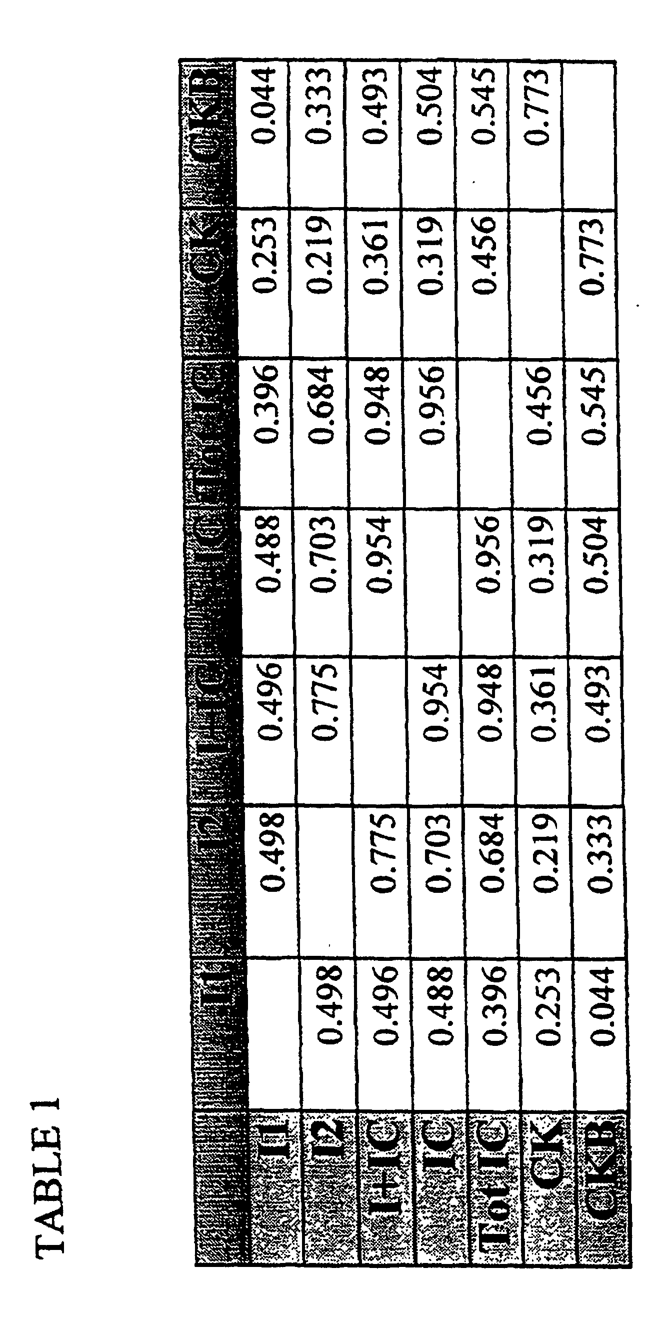

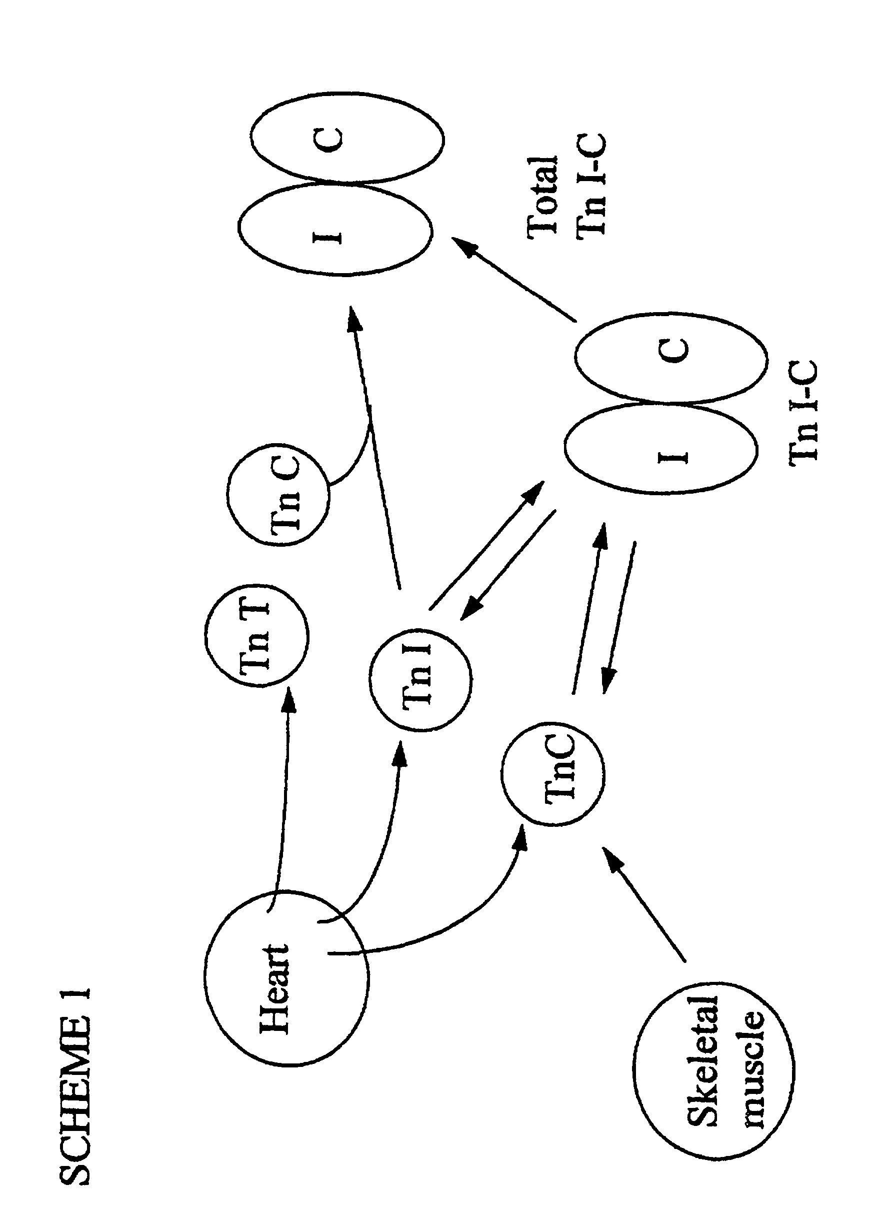

## Claims

1. A diagnostic procedure for the measurement of the heart muscle necrosis in human blood, **characterized by** simultaneous immunochemical measurement of cardiac troponin I-C complex and cardiac troponin I complexable to troponin C in a human plasma or serum sample by using dual labeling of detecting antibodies, or one antibody labeled with a detectable molecule and the added external troponin C labeled with another detectable molecule.

2. A diagnostic method for determination of cardiac muscle necrosis by measuring the level of cardiac specific troponin I-C complex, wherein troponin I is the cardiac isoform, in blood using an immunoassay **characterized by**
(1) incubating a plasma or serum sample with a primary antibody specific to troponin I, C, or I-C,
(2) separation of the formed immunocomplex involving the primary antibody and troponin I-C, and
(3) the measurement of the amount of the isolated immunocomplex with a second labeled antibody (anti-troponin I, anti-troponin C or anti-troponin I-C), so that possible contribution from separate I or C alone are not measured; or alternatively, when the primary antibody is specific only to I-C the formed immunocomplex can be measured also by labeled I-C in a competitive manner.

3. The diagnostic method according to claim 2 **characterized by** the measurement of the signal by a time-resolved fluorescence using single or double labeling method according to claim 1.

4. The diagnostic method according to claim 2, **characterized by** a second measurement of troponin I-C complex comprising of measurement of total troponin I-C formed by externally added troponin C in amounts of 1-300 -fold molar excess to troponin I in patient plasma or serum sample containing cardiac troponin I.

5. The diagnostic method according to claim 4 **characterized in that** troponin C originates from human or animal sources, or is produced by recombinant techniques.

6. The diagnostic method according to claim 4 **characterized by** utilizing an antibody raised against unnatural troponin C, produced by recombinant techniques in such a way that troponin C is able to complex with troponin I.

7. The diagnostic method according to claim 4 **characterized by** the addition of troponin C into the sample into the concentration from 1 microgram to 1 milligram per ml of blood, or alternatively the troponin C is placed into the sampling tube before, or troponin C is added later to the separated serum.

8. The diagnostic method according to claim 2 or claim 4 **characterized by** evaluation of the cardiac muscle necrosis by the ratio of signal with and without addition of troponin C.

9. A diagnostic procedure for the measurement of heart muscle necrosis in human blood **characterized by** the determination of the ratio in accordance with claim 8 using the immunochemical method described in claim 2 except that two different antibodies against troponin I are used in such a way that one of these antibodies has epitope on troponin I at the binding site of troponin C and the other one binds to any other epitope in troponin I, while the measurement is accomplished in the presence and absence of a chelating substance such as EDTA in concentration of 1-50 mM in the serum sample.

10. A diagnostic procedure for the measurement of heart muscle necrosis in human blood **characterized by** the determination of troponin I in a human plasma or serum sample and troponin I-C complex according to claim 2 or 4 and the ratio is utilized to estimate the degree of heart necrosis.

## Patentansprüche

1. Diagnostisches Verfahren zur Messung der Herzmuskel-Nekrose in humanem Blut, **gekennzeichnet durch** gleichzeitige immunchemische Messung von kardialem Troponin I-C-Komplex und kardialem Troponin I, das mit Troponin C komplexierbar ist, in einer humanen Plasma- oder Serumprobe, indem eine Doppel-Markierung von Nachweisantikörpern angewendet wird oder indem ein Antikörper, der mit einem nachweisbaren Molekül markiert ist, und das zugesetzte externe Troponin C, das mit einem anderen nachweisbaren Molekül markiert ist, verwendet werden.

2. Diagnostisches Verfahren zur Messung der Herzmuskel-Nekrose durch Messen des Spiegels von spezifischem kardialem Troponin I-C-Komplex, wobei Troponin I die kardiale Isoform ist, in Blut unter Verwendung eines Immunoassays, **gekennzeichnet durch**
(1) Inkubieren einer Plasma- oder Serumprobe mit einem ersten Antikörper, der für Troponin I, C oder I-C spezifisch ist,
(2) Abtrennen des gebildeten Immunkomplexes, der den primären Antikörper und Troponin I-C umfasst, und
(3) Messung der Menge des isolierten Immunkomplexes mit einem zweiten markierten Antikörper (Anti-Troponin I, Anti-Troponin C oder Anti-Troponin I-C) derart, dass ein möglicher Beitrag von getrenntem I oder C allein nicht gemessen wird, oder dass alternativ, wenn der erste Antikörper nur für I-C spezifisch ist, der gebildete Immunkomplex in kompetitiver Weise auch **durch** markiertes I-C gemessen werden kann.

3. Diagnostisches Verfahren nach Anspruch 2, **gekenn** **zeichnet** durch die Messung des Signals durch zeitaufgelöste Fluoreszenz unter Verwendung eines Einfach- oder Doppel-Markierungs-Verfahrens nach Anspruch 1.

4. Diagnostisches Verfahren nach Anspruch 2, **gekennzeichnet durch** eine zweite Messung von Troponin I-C-Komplex, umfassend eine Messung von Gesamt-Troponin I-C, das **durch** zugesetztes externes Troponin C in Mengen des 1-300-fachen Überschusses zu Troponin I in der Patientenplasma- oder Patientenserumprobe, die kardiales Troponin I enthält, gebildet wird.

5. Diagnostisches Verfahren nach Anspruch 4, **dadurch gekennzeichnet, dass** Troponin C aus humanen oder tierischen Quellen stammt oder durch Rekombinationstechniken produziert wurde.

6. Diagnostisches Verfahren nach Anspruch 4, **gekennzeichnet durch** die Verwendung eines Antikörpers, der gegen unnatürliches Troponin C gerichtet ist, welches **durch** Rekombinationstechniken derart produziert wird, dass Troponin C fähig ist, mit Troponin I zu komplexieren.

7. Diagnostisches Verfahren nach Anspruch 4, **dadurch gekennzeichnet, dass** Troponin C in einer Konzentration von 1 Mikrogramm bis 1 Milligramm pro ml Blut zu der Probe gegeben wird oder alternativ das Troponin C vorher in das Probenröhrchen gegeben wird oder Troponin C später zu dem abgetrennten Serum gegeben wird.

8. Diagnostisches Verfahren nach Anspruch 2 oder Anspruch 4, **gekennzeichnet durch** die Beurteilung der Herz-Muskel-Nekrose **durch** das Verhältnis des Signals mit Zusatz von Troponin C zu dem ohne Zusatz von Troponin C.

9. Diagnostisches Verfahren zur Messung der Herzmuskelnekrose in humanem Blut, **gekennzeichnet durch** die Bestimmung des Verhältnisses gemäß Anspruch 8 unter Verwendung des immunchemischen Verfahrens, das in Anspruch 2 beschrieben ist, außer dass zwei unterschiedliche Antikörper gegen Troponin I derart verwendet werden, dass einer dieser Antikörper ein Epitop an Troponin I an der Bindungsstelle von Troponin C hat und der andere an irgendein anderes Epitop in Troponin I bindet, wobei die Messung in Gegenwart und Abwesenheit einer chelatbildenden Substanz wie z. B. EDTA in einer Konzentration von 1 bis 50 mM in der Serumprobe durchgeführt wird.

10. Diagnostisches Verfahren zur Messung der Herzmuskel-Nekrose in humanem Blut, **gekennzeichnet durch** die Bestimmung von Troponin I in einer humanen Plasma- oder Serumprobe und von Tropin I-C-Komplex nach Anspruch 2 oder Anspruch 4 und wobei das Verhältnis zur Bestimmung des Grad der Herznekrose ausgenutzt wird.

## Revendications

1. Démarche de diagnostic destinée à mesurer dans le sang humain la nécrose du muscle cardiaque, **caractérisée par** la mesure immunochimique simultanée du complexe troponine I-C cardiaque et de la troponine I cardiaque complexable à la troponine C dans un échantillon de plasma ou de sérum humain, au moyen du double marquage d'anticorps de détection ou d'un anticorps marqué avec une molécule détectable et de la troponine C externe ajoutée marquée avec une autre molécule détectable.

2. Méthode de diagnostic destinée à la détermination de la nécrose du muscle cardiaque par mesure du taux de complexe troponine I-C cardiaque spécifique, dans laquelle la troponine I est l'isoforme cardiaque, dans le sang, en utilisant un dosage immunologique, **caractérisée par**
(1) l'incubation d'un échantillon de sérum ou de plasma avec un anticorps primaire spécifique à la troponine I, C ou I-C,
(2) la séparation de l'immunocomplexe formé impliquant l'anticorps primaire et de la troponine I-C, et
(3) la mesure de la quantité de l'immunocomplexe isolé avec un second anticorps marqué (anti-troponine I, anti-troponine C ou anti-troponine I-C), de façon à ce qu'une contribution éventuelle de I ou C séparé seul ne soit pas mesurée; ou selon une autre possibilité, lorsque l'anticorps primaire est spécifique seulement pour I-C, l'immunocomplexe formé puisse être mesuré également par I-C marqué selon une méthode par compétition.

3. Méthode de diagnostic selon la revendication 2, **caractérisée par** la mesure du signal par fluorescence résolue dans le temps, en utilisant une méthode de marquage simple ou double selon la revendication 1.

4. Méthode de diagnostic selon la revendication 2, **caractérisée par** une deuxième mesure du complexe troponine I-C, comprenant la mesure de la troponine I-C totale formée par de la troponine C ajoutée de manière externe en des proportions molaires 1-300 fois supérieures à celle de la troponine I dans un échantillon de plasma ou de sérum du patient contenant la troponine I cardiaque.

5. Méthode de diagnostic selon la revendication 4, **caractérisée par le fait que** la troponine C est d'origine humaine ou animale, ou est produite par des techniques recombinantes.

6. Méthode de diagnostic selon la revendication 4, **caractérisée par** l'utilisation d'un anticorps dirigé contre la troponine C non naturelle, produite par des techniques recombinantes de telle façon que la troponine C soit capable de complexer avec la troponine I.

7. Méthode de diagnostic selon la revendication 4, **caractérisée par** l'addition à l'échantillon de troponine C à une concentration de 1 µg à 1 mg de sang, ou selon une autre possibilité, par le fait que la troponine C est disposée dans un tube à échantillon préalablement ou ajoutée plus tard au sérum séparé.

8. Méthode de diagnostic selon la revendication 2 ou 4, **caractérisée par** l'évaluation de la nécrose du muscle cardiaque par le rapport du signal avec addition de troponine C au signal sans addition de troponine C.

9. Démarche de diagnostic destinée à mesurer dans le sang humain la nécrose du muscle cardiaque, **caractérisée par** la détermination du rapport selon la revendication 8, en utilisant la méthode immunochimique décrite dans la revendication 2, sauf que deux anticorps différents, dirigés contre la troponine I sont utilisés de telle façon qu'un de ces anticorps présente un épitope sur la troponine I au niveau du site de liaison de la troponine C et que l'autre se lie à tout autre épitope sur la troponine I, tandis que la mesure est réalisée en présence ou en l'absence d'une substance chélatante telle qu'EDTA à une concentration de 1-50 mM dans l'échantillon de sérum.

10. Démarche de diagnostic destinée à mesurer dans le sang humain la nécrose du muscle cardiaque, **caractérisée par** la détermination de la troponine I dans un échantillon de plasma ou de sérum humain, et du complexe troponine I-C selon la revendication 2 ou 4 et par le fait que le rapport des deux déterminations est utilisé pour évaluer le degré de la nécrose cardiaque.
